# EUROPEAN PATENT APPLICATION

(11) **EP 4 850 706 A1**
(43) Date of publication of application: **09.12.2026**
(21) Application number: 25181069.3
(22) Date of filing: 05.06.2025
(51) Int. Cl.: A61L 31/12, A61L 31/14, A61B 34/00, A61B 1/00

(54) **MICROROBOT**

(71) Applicant: ETH Zurich, 8092 Zurich (CH)
(72) Inventor: SCHÜRLE-FINKE, Simone, 8127 Forch (CH); CESAROVIC, Nikola, 8050 Zürich (CH); YAN, Yimo, 8048 Zürich (CH)

(57) **Abstract**

The invention concerns a microrobot that is suitable for targeted drug delivery. The microrobot is biocompatible. It comprises a plurality- for example a large number- of magnetic particles. The microrobot is magnetically anisotropic in that the energetically most favorable state depends on an orientation of the microrobot in the external magnetic field, thus a direction of the external magnetic field relative to a magnetic main axis of the microrobot. In addition to being magnetically anisotropic, the microrobot also has an overall anisotropic shape. The combined magnetic and shape anisotropy may be used to steer the microrobot to a no-reflow zone near a vascular obstruction and to assist the drug delivery to the vascular obstruction.

## Description

### FIELD OF THE INVENTION

The invention is in the field of medical technology. More particularly, it relates to devices for supporting the delivery of a therapeutic agent to an occluded site in a vessel, in particular to devices for assisting the treatment of vascular obstructions, especially microvascular obstructions (MVO). It also relates to a kit of pats with such device, a method of manufacturing such devices, as well as to a therapeutic agent for use in a method that uses the devices for administering the therapeutic agent, and to a medical method.

### BACKGROUND OF THE INVENTION

Ninety percent of clinical drug development fails despite carefully implemented evaluation strategies. Analysis of clinical trial data indicates that approximately 57% of failures are attributed to insufficient efficacy, and systemic delivery, the most common route of drug administration, causes unacceptable levels of toxicity in another 30% of trials (JAMA Intern Med 12, 1826-1833 (2016)). Efforts to transform some promising therapeutic agents into effective treatments concentrate on enhancing drug solubility, controlling release profiles, expanding therapeutic activity, optimizing pharmacokinetics, and augmenting stability (Nat Biomed Eng 5, 951-967 (2021)).

Specifically, drug delivery systems (DDS) such as nanoparticles, microparticles, and pH-responsive capsules play pivotal roles in this advancement by significantly improving drug stability and controlling pharmacokinetics. However, current DDS are still insufficient in ensuring precise delivery of therapeutics to anatomically challenging disease sites. Precise delivery is critical for mitigating toxicity and enhancing targeted efficacy.

In recent years, microrobots have attracted significant interest due to their enhanced controllability and substantial potential as innovative DDS in clinical settings. Multiple medical functions have been demonstrated, including cancer therapy, thrombolytic therapy, hyperthermia, and embolization. The active navigation capabilities of microrobots overcome the limitations of systemic delivery and local diffusion by enhancing the targeted delivery of concentrated therapeutic and/or diagnostic agents to disease sites. Nevertheless, multiple challenges must be addressed before microrobotic devices can be clinically implemented, including ensuring the biocompatibility of materials and understanding the dynamic behavior of microrobots. Additional issues include scalable production, cost-effectiveness, operational feasibility, and device acceptance by clinicians. Currently, there are no commercially available microrobots that effectively address these challenges.

A situation where drug delivery is particularly challenging is the occurrence of MVO, also known as 'no-reflow' phenomenon. It occurs when small vessels become occluded, impeding tissue perfusion. To date, clinical trials focusing on thrombolytic therapy for MVO, such as the INFUSE-AMI trial (Eur. Heart J. Acute Cardiovasc 7 339-347 (2018)), have not been successful. A potential reason for these failures is the challenge of effectively delivering thrombolytic agents via intra-coronary or systemic administration. The drugs partially or entirely bypass the regions of MVO, which exhibit higher fluidic resistance compared to unobstructed vascular networks.

### SUMMARY OF THE INVENTION

It is an object of the present invention to overcome disadvantages of the prior art and to provide approaches for effectively delivering a therapeutic agent to a specific location, especially to an obstructed blood vessel, for example of a small blood vessel such as a so-called microvessel (arteriole, capillary, metarteriole, venule).

These and other objects are achieved by a microrobot, a kit of parts, a manufacturing method, a thrombolytic agent for use in a method, and a method as defined in the claims.

According to an aspect of the invention, the invention concerns a microrobot that is suitable for targeted drug delivery. The microrobot is biocompatible. It may comprise materials that are not toxic, that are suitable for medical applications and that may be accepted and approved for such medical applications, for example approved in the EU at the application date of the present patent application. It comprises a plurality- for example a large number- of magnetic particles. A magnetic particle in the sense of the present text is a particle of a material that is magnetic (ferromagnetic or ferrimagnetic) at 37°C, i.e., a material that exhibits so-called magnetic order (or 'long-range magnetic order') and has a bulk phase transition temperature (Curie temperature) of more than 37°. Depending on the size, such magnetic particles may exhibit superparamagnetic behavior because the magnetization direction within each particle changes over time due to thermal fluctuation.

The microrobot will especially be resorbable, by the human (or animal) organism. This means that the material of the polymer matrix may be such as to decompose over time, for example on a time scale between a few hours and a few years, in the human (or animal) body, and the magnetic particles are capable of being resorbed, too.

The microrobot is magnetically anisotropic in that the energetically most favorable state depends on an orientation of the microrobot in the external magnetic field, thus a direction of the external magnetic field relative to a magnetic main axis of the microrobot.

In addition to being magnetically anisotropic, the microrobot also has an overall anisotropic shape. The fact that the microrobot has an overall anisotropic shape means that its shape is distinct from spherical not only due to random fluctuations, but systematically. Especially, the shape may be generally the shape of an ellipsoid, especially a prolate spheroid (i.e., an ellipsoid with the two shorter axes having approximately equal lengths). If the shape is approximated by an ellipsoid, for example a prolate spheroid, the long axis of the ellipsoid is substantially, for example by at least 20%, larger than an average of the shorter axes.

Especially, a main axis (longest axis if the shape of the microrobot is approximated by an ellipsoid) of the shape may coincide with the magnetic major axis.

A longest extension of the microrobot may be between 1 µm and 50 µm or between 3 µm and 35 µm, in particular between 4 µm and 25 µm, for example between 5 µm or 6 µm and 15 µm. If the shape of the microrobot is approximately the shape of an ellipsoid (especially a prolate spheroid) a length of the shorter axes may be between 20% and 80%, especially between 30% and 70% of a length of the longest axis.

If the size (longest extension) of the microrobot is between about 7 µm and 14 µm, the size is comparable to the size of blood cells. This can be advantageous for many applications: on the one hand, the size is such that the microrobots can progress to even the finest blood vessels so as to be helpful in the treatment of MVO. On the other hand, the size has been found to be large enough for the ensemble of microrobots to advance with sufficient velocity and to generate sufficient convection.

The combination of the magnetic anisotropy with the shape anisotropy has the following effect: in a rotating magnetic field, with sufficiently small rotation frequency, the magnetic anisotropy will cause the microrobot to rotate, too, if the microrobot is free to rotate, for example by being suspended in a liquid environment, such as in blood plasma. Due to the shape anisotropy, such rotation of the microrobot may firstly assist locomotion of the microrobot. Especially, if the microrobot is interfacing with a wall of a vessel containing a liquid, such as a blood vessel in the human body, the rotation may for example cause the microrobot to translate along a wall of the vessel due to friction. Secondly, the shape anisotropy may produce convective transport of the liquid around it if the microrobot rotates, due to effects related to ferrohydrodynamic coupling.

Both these effects that result from the combination of magnetic anisotropy and shape anisotropy may be helpful for targeted drug delivery. The improved locomotion may assist a swarm of microrobots to reach a location where the drug is to be delivered, for example a thrombus. This may be used in different ways. Firstly, locomotion of a swarm of microrobots causes, by hydrodynamic coupling, a flow of liquid in which they are immersed, and this may be utilized for the transport of a therapeutic agent dispensed into the liquid. Additionally or as an alternative, in embodiments, the microrobots themselves may contain a therapeutic agent, which agent is released when the target is reached. Secondly, in addition to the effect of the movement of the microrobots, also the convection caused by the rotation assists the drug delivery process. To sum up, the propulsion caused by the rotating microrobots disrupts the static flow within a region that otherwise would have no flow or only little flow, as for example typically present near a thrombus. The propulsion will generate a volumetric net flow which significantly enhances the penetration and distribution of therapeutic agents to the target site, overcoming the limitations typically imposed by passive diffusion processes.

The locomotion and convection generating capacity of the microrobot will significantly assist the therapeutic effect of a therapeutic agent in a region with weak circulation of blood (or other body fluid). For the situation of MVO, simulation results indicate that following a 1-minute administration of the therapeutic agent (active pharmaceutical ingredient, API), adequate drug concentration cannot be achieved in the no-reflow region by passive diffusion alone. The occlusion leads to elevated resistance in the vessel which forces the majority of the therapeutic to bypass the no-reflow region. Consequently, prolonged administration of thrombolytic agents becomes the only choice for the lysis of microthrombi in these regions. However, this approach increases the risk of excessive bleeding which significantly impacts patient outcomes and, in severe cases, leads to mortality. Due to significantly reduced or absent blood flow induced by MVO, successful navigation of microrobots into these poorly perfused areas will overcome the constraints imposed by the flow profile.

The matrix material may be chosen so as to be resorbed by the human organism within a few hours through a few years. A suitable matrix material is Dextran. Dextran-based microrobots, for example, will typically be resorbed withing about 2-8 months. Other possible matrix materials include other biocompatible, resorbable polymers, especially polylactides (PLA), a polyglycolic acid (PGA), polyethylene glycol (PEG) or copolymers of the according monomers, including poly(lactic-co-glycolic) acid (PLGA). Generally, there exist biodegradable materials that are employed in commercial microspheres, and these may for example be used as the matrix material.

The magnetic particles may be nanoparticles, having an average particle size (average diameter) of less than 150 nm, for example less than 100 nm, especially between 5 nm and 70 nm or between 20 nm and 50 nm. The size of the magnetic particles may be small enough for their ensemble to exhibit superparamagnetic properties (at 37°C). A material of the magnetic particles may be iron oxide, for example (Fe₃O₄) or ( γ-Fe₂O₃). Generally, every magnetic material that is biocompatible and has a Curie temperature above 37°C is in principle suitable.

Resorption of the magnetic particles may be achieved via the liver and may take place some time after resorption of the matrix material. The acceptability of magnetic nanoparticles of for example Fe₃O₄ or γ-Fe₂O₃, but also of other materials has been confirmed for different medical applications, or example in the context of applications for diagnostics or cancer treatment
The microrobots may for example be provided in the form of a physiologically acceptable solution, in particular aqueous solution, with a large number of the microrobots being suspended therein. It is also possible that they are provided as a powder to be suspended in a carrier liquid immediately before therapeutic intervention.

A kit of parts to be used for therapeutic intervention and containing the microrobots may in addition to the microrobots comprise a therapeutic agent to be delivered together with the microrobots and/or equipment for the delivery. Such equipment may for example comprise a catheter system, possibly comprising a balloon catheter that may temporarily occlude the vessel to prevent any backflow of the microrobots and the therapeutic agent through the vessel into which they have been dispensed. Equipment for carrying out a therapy using the microrobots may in addition comprise a source of a rotating magnetic field to be applied to a treatment zone of the patient as well as equipment for navigation, for example comprising imaging equipment.

Magnetic navigation setups are under development and are also commercially available from different suppliers, such as, at the priority date of the present application, from 'Stereotaxis', from 'Nanoflex robotics', from 'Euphratesvascular' ('Pulse Therapeutics') or from 'Bionaut Labs'.

The treatment zone in the present text is the region of the patient's body that is affected by the treatment. It will include the location where the treatment is to have its effect, for example the location of the embolus/emboli in case of MVO, as well as a region in this location's vicinity. It may further include the location where the microrobots are dispensed into the vessel as well as the region between this location and the location of the embolus/emboli.

For a therapy of a human patient or a (non-human) animal patient, the following steps may be applied:
- providing a plurality of microrobots as described and defined in the present text, i.e., microrobots that comprise a plurality of magnetic particles and a polymer matrix embedding the magnetic particles, wherein the magnetic particles and the polymer matrix are biocompatible and resorbable, wherein the microrobots are magnetically anisotropic, and wherein the microrobots have an overall anisotropic shape;
- administering the microrobots to a vessel in a treatment zone of the patient; and
- applying a rotating magnetic field to the treatment zone, so as to cause the microrobots to undergo a synchronous rotational movement.

Generally, the embodiments and variants of microrobots described in the present text can be used for the method. Properties described in the present text for characterizing the microrobots and the method of their manufacture may also characterize the method and the thrombolytic agent for use in the method.

If the therapy is a therapy of a thrombotic condition, then administration of the microrobots will be administration to a blood vessel, and the therapy will comprise the further step of administering a thrombolytic agent to the blood vessel. This administration of the thrombolytic agent can be done via any route suitable for the thrombolytic agent. In many embodiments, the route directly into the vessel (as opposed to orally or via another route, is preferred, wherein the access used for the administration of the microrobots can be used, too, especially a catheter-based access. Thus, administration may comprise administering the thrombolytic agent directly into the blood, using a same device (catheter, syringe) or a different device as for the administration of the microrobots.

It is also possible that the microrobots comprise the thrombolytic agent, and the administration of the thrombolytic agent is achieved by the administration of the microrobots.

The rotating magnetic field applied for the treatment may especially be a low frequency rotating magnetic field, having a frequency that is smaller than a step-out frequency being a frequency beyond which the microrobots suspended in the body liquid (for example blood plasma if the microrobots are administered to a blood vessel) cannot synchronize with the rotating magnetic field. The step-out frequency depends, in addition to depending on properties of the body liquid, on a size and shape of the microrobots and on the achievable magnetic moment of the microrobots given the strength of the applied magnetic field and can be determined experimentally. In embodiments, the frequency of the rotating magnetic is between 5 Hz and 30 Hz, especially below 25 Hz, for example less than 20 Hz. It is not excluded, though, that a rotation frequency of the field is higher than the step-out frequency, for example an integer multiple thereof.

The present invention also concerns a thrombolytic agent for use in a therapeutic method as described herein.

Since the effect of the microrobots is primarily mechanical (locomotion/transport, convection generation), this will work for any agent having a thrombolytic effect. In addition or as an alternative, administration may comprise the use of the microrobot as a vehicle to transport the thrombolytic agent by using the microrobots to carry the thrombolytic agent, such as by causing the thrombolytic agent to be chemically or physically bound to the microrobot and to cause the bond to be released when the target site has been reached, by the thrombolytic agent reacting to a change of environment when the thrombus has been reached (that might depend on the kind of thrombus or for example by causing a condition to be changed at that stage).

A method of manufacturing microrobots may comprise the following steps: Initially, a flowable compound of magnetic particles, especially nanoparticles, suspended in a pre-matrix material, is provided. The pre-matrix material may comprise a monomer or oligomer (thus a prepolymer), and/or a (flowable) polymer. The method may comprise the further step of dispersing a portion of the flowable compound to yield droplets, for example suspended in a carrier liquid with which the flowable compound is not miscible. In this, a volume of the droplets may for example essentially correspond to a volume of the microrobots to be manufactured. The method may further comprise exposing the droplets to a magnetic field while the pre-matrix material is still flowable. The magnetic field may for example be a rotating magnetic field, for example with a comparably small rotation frequency, such as a rotation frequency of less than 30 Hz, less than 20 Hz or even 10 Hz or less, with a possible minimum rotation frequency being 1 Hz. While the magnetic field impinges, the pre-matrix material is caused to harden, for example by a polymerization process (if the pre-matrix material comprises prepolymer) and/or by cross-linking.

In this, the hardening process may be driven by any suitable measure, such as chemically, for example by a polyaddition reaction, by providing a substance that causes the hardening process (for example, if the droplets are suspended in a carrier liquid, an additive of the carrier liquid and available for the flowable compound via the droplet-carrier-liquid interface), and/or by a suitable energy input, for example UV irradiation and/or heating, and/or in any other suitable way.

In embodiments, the carrier liquid is a hydrophobic substance, and the flowable compound is hydrophilic, whereby the mixture of the droplets and the carrier liquid is a water-in-oil emulsion. Depending on the pre-matrix material, it would also be possible to provide the flowable compound as the hydrophobic phase and the carrier liquid as the hydrophilic phase, thus to use an oil-in-water emulsion.

The described manufacturing process uses the tendency of the magnetic (nano-) particles to arrange themselves in an energetically optimized configuration in the externally applied magnetic field, thus in a configuration in which they are arranged basically aligning with the magnetic field. Because the magnetic nanoparticles are suspended in the pre-matrix material while the pre-matrix material is still flowable, it is possible for them to re-arrange from an initial, spherical configuration to a configuration in which they are extended along the magnetic field. This causes the droplet as a whole, with the magnetic particles suspended in the flowable pre-matrix material, to obtain an elongate/stretched shape. Thereby, the magnetic particles serve as structural backbone of the microrobot produced. The pre-matrix material will harden to become the matrix material while the droplet has this elongate shape, thereby resulting in a structurally and magnetically anisotropic microrobot.

The manufacturing method may in embodiments comprise the additional step of PEGylating the microrobot after the step of hardening. PEGylation is per se known. It has been found experimentally for microrobots with Dextran as the matrix material that a PEGylation step is effective in enhancing the biocompatibility and stability and in minimizing the likelihood of inflammation in physiological environments.

### BRIEF DESCRIPTION OF THE DRAWINGS

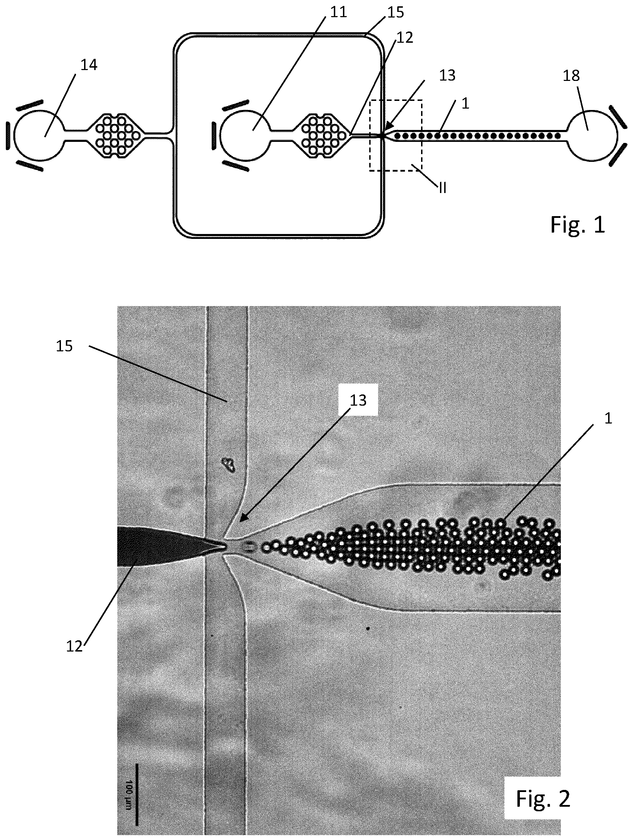

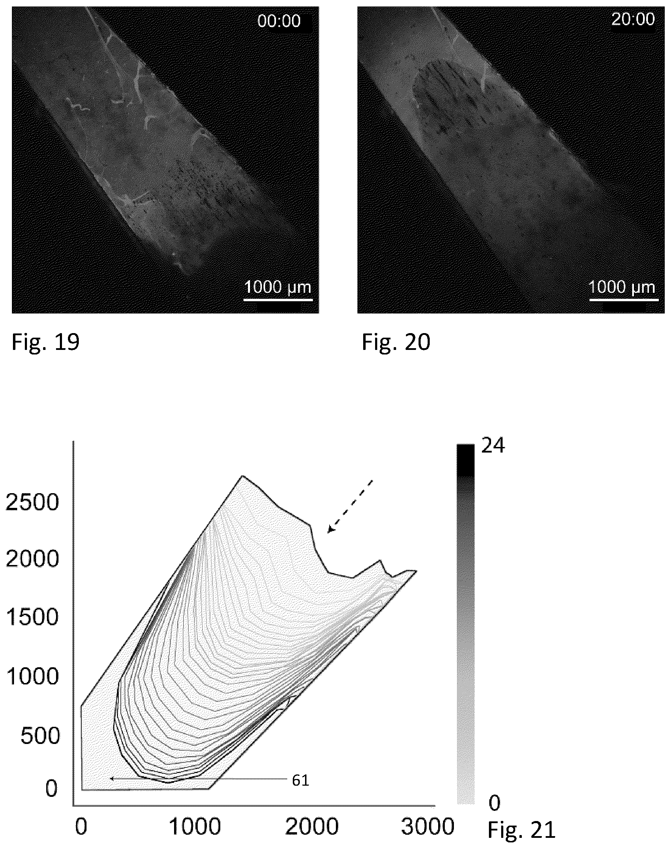

Hereinafter, embodiments of the present invention are described referring to drawings. In the drawings, same reference numbers denote same or analogous elements. The drawings are, with the exception of the photographs shown in Figs. 2, 4, 8, 9, 19 and 20 and the representation in Fig. 21, mostly schematical and not to scale. They show:
- Fig. 1: a system used in a process of manufacturing the microrobots;
- Fig. 2: a photograph of a microscope image;
- Fig 3: a scheme of the manufacturing process;
- Fig. 4: a scanning electron microscopy image of a microrobot;
- Fig. 5: a size distribution of the major axis and the minor axes of microrobots;
- Fig. 6: the results of a measurement of an average velocity of anisotropic microrobots and isotropic particles of a same volume, respectively, in a rotating magnetic field;
- Fig. 7: a sketch illustrating how a rotating magnetic field can cause locomotion of the microrobots;
- Fig. 8: a time-lapse image of a branched in-vitro system with anisotropic microrobots drifting in the flow direction, bypassing a non-flow region;
- Fig. 9: a time lapse image of the branched in-vitro system when a rotating magnetic field is applied, with the anisotropic microrobots being guided into the no-reflow region;
- Fig. 10: a drawing illustrating the principle of a therapeutic treatment;
- Figs. 11-16: a sequence of steps of a clinical workflow for the therapeutic treatment for eliminating a MVO;
- Fig. 17: results of an in-vitro measurement, namely a normalized lysis of fibrin embolus material as a function of the time under different conditions;
- Fig. 18: results of an in-vitro measurement namely a normalized lysis of porcine blood clots as a function of the time under different conditions;
- Fig. 19-20: photographs of an in vitro thrombolysis process in a Y-branched system comprising a porcine blood clot, at an initial stage and after 20 minutes of the treatment, respectively; and
- Fig. 21: image recognition derived curves showing the periphery of an embolus at different points in time, evidencing a progressive formation of an inward-concave valley.

### DESCRIPTION OF THE PREFERRED EMBODIMENTS

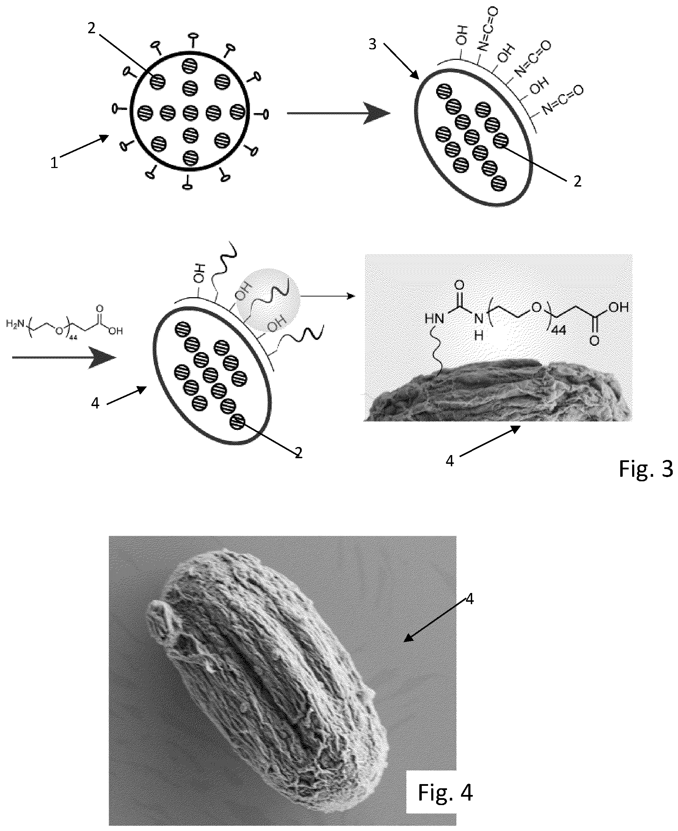

**Figure 1** schematically illustrates an embodiment of a system used in a process of manufacturing a plurality of microrobots, and **Figure 2** shows a photograph of a microscope image taken from the detail II in Fig. 1. **Figure 3** shows a scheme of an example of the manufacturing process.

In the depicted embodiment, an aqueous phase comprising Dextran polysaccharide chains (*Mₙ*= 6 KDa) as the matrix material and encapsulated magnetic nanoparticles (MNPs) is conveyed from an aqueous phase storage 11 via an aqueous phase line 12 to a flow-focusing droplet-based microfluidic microdroplet generating stage 13. The magnetic nanoparticles are 10 nm iron oxide nanoparticles (supplier: Ferrotec, EMG 700 series).

In the system of Fig. 1, a hydrophobic phase, for example of a commercially availabe Hydrofluoroether (HFE) oil (3M^{™} Novec^{™} 7500 Engineered Fluid, containing a surfactant and being supplied by RAN Biotec) is also conveyed to the microdroplet generating stage 13, from a hydrophobic phase storage 14 via a hydrophobic phase line 15. In the microdroplet generating stage 13, a microdroplet-in-oil emulsion is generated, with the aqueous phase being the dispersed phase and the HFE being the continuous phase. The microdroplets 1 comprise the Dextran as primary hydrogel and comprise the encapsulated magnetic nanoparticles 2 (schematically shown in Fig. 3, top left panel) and have thus magnetic properties.

In a subsequent step, the water-in-oil precursor magnetic droplet is caused to undergo crosslinking via a polyaddition reaction, with toluene diisocyanate being added to serve as a crosslinker. In the depicted embodiment, this reaction is initiated by the toluene diisocyanate at the droplet interface.

An external magnetic field, in an embodiment produced by a Rotating Halbach Cylinder device, is applied while the polyaddition reaction takes place. The rotating magnetic field has a low frequency of less than 10 Hz. Such field causes, via dipolar interactions, the MNPs to be aligned into elongate bundle configurations within the precursor droplet. Because of the flowability of the Dextran precursor liquid, this causes the precursor droplet as a whole to become elongate and aligned with the magnetic field, as sketched in Fig. 3, top left and right panel. The polymerization and cross-linking processes taking place within the precursor droplet cause the droplet to become dimensionally stable, with the nanoparticles being a structural backbone within the crosslinked dextran polymer network that serves as the dextran polymer matrix. The result is an ellipsoid-shaped microrobot.

For comparison, a same process was carried out in a magnetic field rotating at higher frequencies of substantially more than 10 Hz. Under such conditions, spinning MNP bundles generate localized fluidic vortices, resulting in the formation of disk patterns in the droplet but not to an overall elongate ellipsoid shape.

Subsequently, in embodiments, the ellipsoid-shaped microrobots produced by the hereinbefore described steps are subject to a PEGylation step (Fig. 3, bottom left panel), to leverage unreacted isocyanate groups on the surface of the microrobots. For example, unreacted isocyanate groups can be utilized for surface functionalization by covalently attaching amine groups from Amine-PEG-Carboxyl (NH2-PEG-COOH). This modification strategy leads to an enhancement of the microrobot's biocompatibility and stability and to a minimization of the likelihood of inflammation in physiological environments.

The presence of unreacted isocyanate groups prior to the PEGylation step was experimentally confirmed, for the microrobots with the Dextran matrix, by Cyanin3-Amine and Cyanin3-Azide, as isocyanate groups specifically react with amine groups. Comparative experiments were used to demonstrated that the amine group exhibited a fluorescence intensity of Cyanin3 that was a hundred times higher than the azide group, thereby confirming the existence of unreacted isocyanate groups. Fourier transform infrared (FTIR) spectrometry confirmed the presence of Amine-PEG functional groups after the PEGylation step.

The shape, regularity and size of microrobots produced by this exemplary process were confirmed by Scanning Electron Microscopy (SEM), Focused Ion Beam (FIB) imaging (**Figure 4**) and Energy-Dispersive X-ray spectroscopy imaging, the latter also confirming a homogeneous coverage of carbon and oxygen on the surface of the iron constituents. In particular, the iron was shown to be distributed over a whole cross section (along a plane through the long axis of the microrobot) of the microrobot. This homogeneous distribution suggests that the dextran network effectively stabilizes the bundles' configuration of the MNPs, thereby reinforcing the structural integrity of the microrobots.

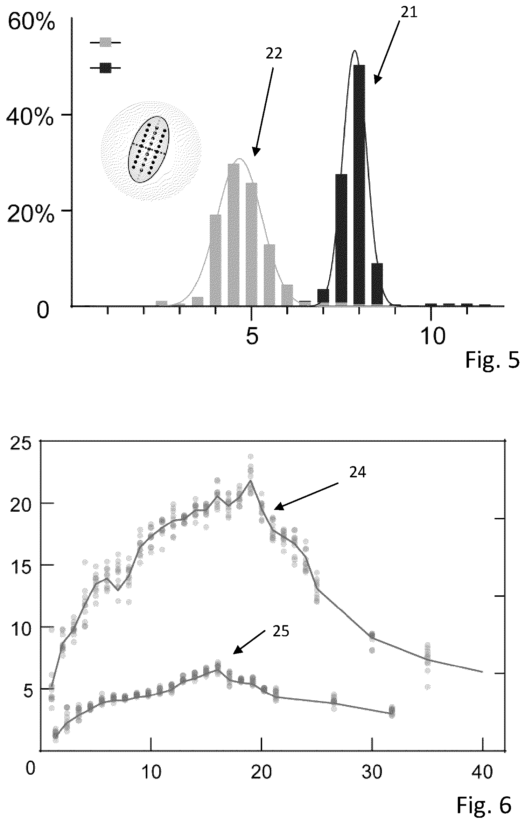

In addition, particle image analysis of microscopy images revealed a narrow distribution of the major and minor axes of the microrobots, indicating a high degree of uniformity in their shape and size. **Figure 5** illustrates the size distribution in micrometers, i.e., the occurrence (in percent) as a function of the particle size in micrometers, with the higher peak 21 attributed to the major axis and the lower peak 22 to the minor axis, confirming an average robot length of 8 µm and an average robot width (extension along the minor axis) of slightly more than 4 µm. The size was measured using an image analysis software ('Fiji') directly determining the particle dimensions.

The PEGylated microrobots exhibit negligible bio-adhesion to human microvascular endothelial cells (HMEC), as evidenced by their experimentally confirmed unimpeded traversal across the HMEC surface without a reduction in microrobot number. The lack of bio-adhesion mitigates the risk of potential vascular occlusion due to cellular adhesion. Also, upon release of the magnetic field, the microrobots disperse in the liquid in which they are suspended. This property prevents potential vascular blockage by the microrobot aggregates. CCK-8 assays reveal that HMECs maintain over 90% viability following a 24-hour exposure to 125 µg/ml of microrobots, with an observed increase in viability beyond 100% after 48 hours, substantiating the low cytotoxic nature of the microrobots. For the unmodified (not PEGylated) microrobot, a measured negative surface charge of approximately -26.4 mV indicates its colloidal stability. Conversely, the PEGylated microrobot displays a marginally higher negative charge of -29.03 mV, attributable to the amino group's replacement with carboxyl groups. Upon deprotonation, these groups transition from COOH to COO-, augmenting the inter-microrobot electrostatic repulsion and thereby increasing the zeta potential's magnitude and hence the colloidal stability.

Additionally, the favorable coagulation properties of the microrobots were confirmed by incubating varying concentrations with porcine blood and measuring key coagulation parameters: prothrombin time (PT), activated partial thromboplastin time (aPTT), thrombin time (TT), and fibrinogen levels. Results demonstrated that the coagulation times and fibrinogen levels were statistically comparable to those observed in the phosphate-buffered saline (PBS) control group, indicating that the microrobots do not instigate clotting cascade activation. The hemocompatibility of the microrobots was further evidenced by a negligible hemolysis rate, reinforcing their suitability for use in blood environment.

In an in vivo pilot study, the microrobots' biosafety was also evaluated in a healthy pig model through intracoronary infusion at varying concentrations (10mg and 20mg).

Initial results demonstrated no adverse effects. ECG signal remained stable, indicating the absence of myocardial injury. These findings suggest that intracoronary microrobot administration is safe at these doses and does not induce detectable physiological disturbances in the pig model, demonstrating potential of future studies evaluating preclinical safety and efficacy.

Further characterization of the microrobots produced by the hereinbefore described process comprises an investigation of the locomotion capability under a rotating magnetic field (RMF). For targeted thrombolysis in MVO, microrobots administered intravascularly possess the capability to navigate along blood flow and navigate into no-reflow regions that are typically inaccessible to passive drugs.

It was observed that the hereinbefore described programming of the magnetic supradomain within the dextran hydrogel body (by subjecting the precursor droplets to a slowly rotating magnetic field during the cross linking process), the average velocity of the thus anisotropic microrobot is nearly five times higher than that of a homogeneous microrobot without such programming. This is shown in **Figure 6****.** The dots in Fig. 6 each represents a measured average velocity (in µm/s) in function of the magnetic field actuation frequency (in Hz) for a microrobot manufactured as explained above (upper measurement curve 24) and for a comparative example being a homogeneous magnetic microparticle of similar size but without the anisotropy induced by the magnetic field acting during cross linking (lower measurement curve 25). Darker shadings are representative of a plurality of measurements with similar average velocity values, leading to an overlap between measurement dots. The lines for each measurements curve connect the averages for each of the applied actuation frequencies. The measurements were carried out by tracking analysis using the above-mentioned image analysis software 'Fiji'.

As becomes clear from Fig. 6, the average velocity is nearly five times as high for the microrobots according to the present invention compared to the homogeneous (and thus isotropic) magnetic particles.

Also, for Fig. 6 it is apparent that the velocity increases approximately linearly as a function of the actuation frequency up to a specific step-out frequency of about 18 Hz, whereafter there is a sharp decrease. This is attributed to the fact that beyond this frequency, the microrobot can no longer synchronize with the external magnetic field, causing its velocity to drop dramatically. In other words, a rotation frequency of the microrobot will correspond to the actuation frequency if the actuation frequency is below or at the step-out frequency, whereas for higher frequencies the rotation frequency is significantly smaller than the actuation frequency.

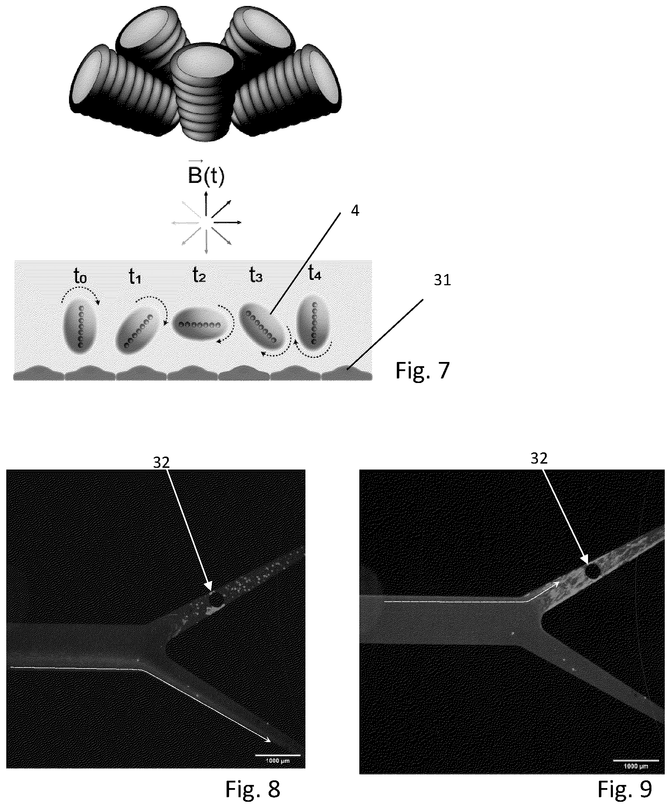

The enhancement in velocity of the anisotropic microrobots compared to the isotropic particles is attributed to the superior magnetic shape anisotropy from both, the overall ellipsoid shape and the multi-bundled structure of magnetic supra-domain. The pronounced structural and magnetic anisotropy improves the microrobot's responsiveness to external magnetic fields, thereby enhancing its locomotion capabilities. This increased velocity and controllability within physiological environments will significantly enhance its efficacy for targeted and convectionenhanced drug delivery. This is sketched in **Figure 7** illustrating that a rotating magnetic field **B**(t) (with coils generating such rotating magnetic field being sketched in the upper part of Fig. 7) may cause the microrobots 4 to rotate. If a microrobot happens to be near a tissue structure 31, such as a wall of a vessel, the rotational movement causes the microrobot to roll along such tissue structure, leading to the increased locomotion as observed in Fig. 6. In Fig. 7, t₀, ... t₄ denote consecutive points in time, with one microrobot 4 shown at these points in time.

The observed locomotion properties can be used to steer the microrobots into the no-reflow region. Experiments demonstrated the locomotion of anisotropic microrobot into the no-reflow region under physiological flow conditions. The measurements were carried out in 2 wt. % polyvinylpyrrolidone (PVP) solution within a Y-junction microfluidic microsystem. One branch of the channel was obstructed using a PDMS pillar to simulate the no-reflow region. In the absence of externally applied magnetics field, the anisotropic microrobots drifted with the flow direction, bypassing the no-reflow area (**Figure 8**). Fig. 8 shows a time-lapse image of the microfluidic microsystem, with the upper branch following the Y-junction obstructed by the PDMS pillar 32, visible as round dot. Fig. 8 shows the anisotropic microrobots drifting in the flow direction and bypassing the non-reflow region, see the bright region immediately above the dashed arrow. However, with the application of a RMF at a frequency of 10 Hz, the microrobots were capable of moving against the boundary flow and successfully penetrated into the no-reflow region (**Figure 9**, see the bright region immediately above the dashed arrow). It could be observed that under the external magnetic field, the microrobots were steered toward the wall adjacent to the no-reflow area. This path allowed the microrobots to avoid high fluid drag force and effectively enter the no-reflow region.

This strategy was corroborated by integrating simulation results with Particle Image Velocimetry (PIV) analysis, which indicated that the low flow velocities near the wall adjacent to the no-reflow region resulted in reduced fluidic drag on the microrobots. Consequently, this reduction in drag allowed the microrobots to more easily overcome the constraints imposed by the surrounding flow, thereby facilitating penetration of microrobot into the no-reflow region.

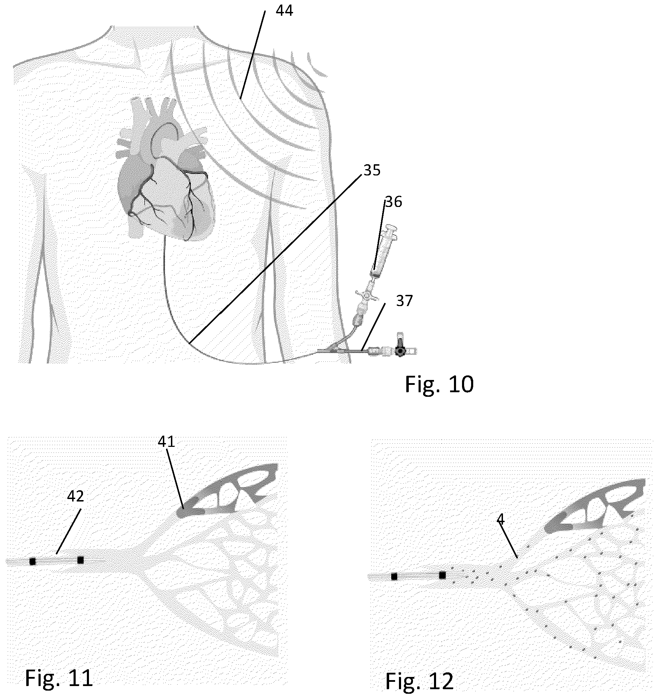

**Figure 10** illustrates the principle of a therapeutic treatment. A catheter 35 is advanced towards the target site in the human body, for example a coronary vessel. A microrobot solution 36 comprising the microrobots as well as a therapeutic agent 37 are dispensed via the catheter, and a time-varying magnetic field 44 is applied to the target site.

More concretely, in an event of MVO at a location ("target site") in a human body, according to one possibility a therapeutic treatment is performed as follows: initially, a balloon catheter is positioned at the pre-arteriole within the coronary artery and guided towards the target site using an imaging technique, such as fluoroscopy. Following this, balloon angioplasty is performed to temporarily occlude the blood vessels, facilitating the controllable administration of the microrobots. A large number of the microrobots is then introduced through the catheter and navigated to the lesion area using external magnetic fields. Once they have reached the occluded area, the microrobots are actuated to perform their intended therapeutic functions, such as targeted drug delivery and possibly mechanical interventions to remove the obstruction.

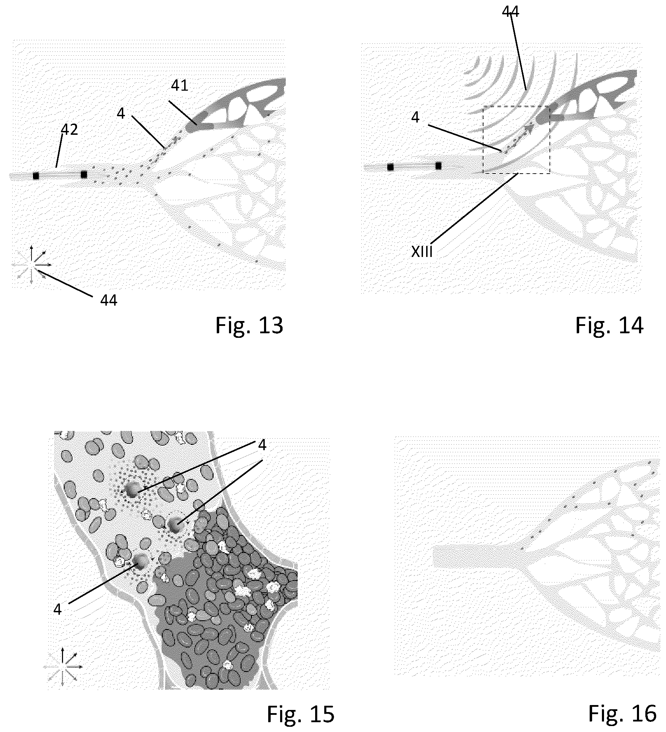

**Figures 11** - **16** show a sequence of steps of a clinical workflow for the therapeutic treatment for eliminating a MVO 41. Step I as illustrated in Fig. 11 comprises positioning a catheter-balloon 42 in a pre-arteriole. Step II (Fig. 12) comprises the catheter-based introduction of microrobots 4 as well as of a therapeutic substance, for example in a liquid state. Step III (Fig. 13) includes the navigation of the microrobots 4 into the no-reflow-region adjacent the MVO 41 using a rotating magnetic field 44. Step IV (Fig. 14) comprises the activation of the therapeutic substance by the microrobots. In the depicted embodiment, this activation comprises the generation of a convective flow by the microrobots 4. The convective flow is generated in that the microrobots rotate under the influence of the rotating magnetic field 44 while immersed in the blood into which the therapeutic agent had been dispensed. Fig. 15 schematically shows a detail of region XIII in Fig. 14 schematically illustrating that the microrobots 4 act to enhance the flow, the dashing around the microrobots indicating the presence of the therapeutic agent. Fig. 15 also illustrates blood cells and platelets. Fig. 16 illustrates step V: retrieval of the catheter after recanalization has been achieved.

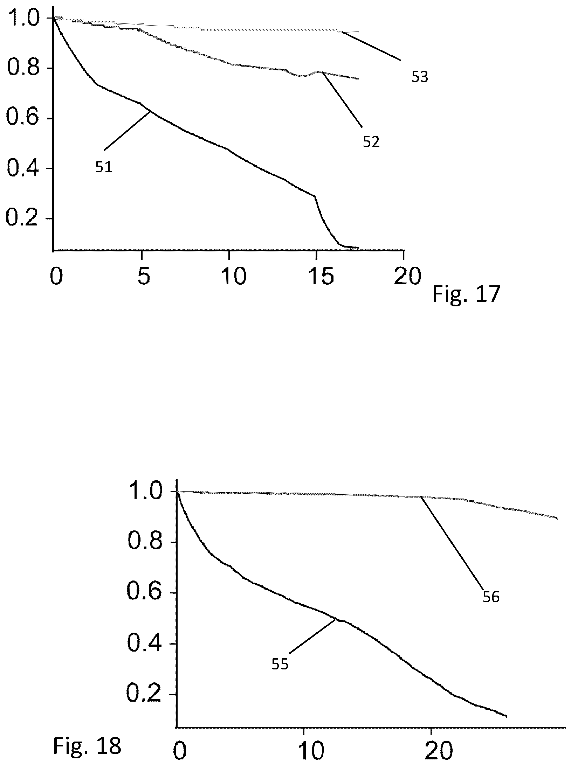

To evaluate the performance of the microrobots, an in vitro experiment was carried out to assess its thrombolytic capabilities on a fibrin embolus within the above-mentioned Y-junction microfluidic microsystem. The experimental setup involved the obstruction of one channel branch with an embolus to simulate the no-reflow region. The microrobot concentration used in the study was 0.5 mg/ml. To ensure uniformity and accuracy, initially an artificial fibrin embolus was fabricated for in vitro lysis experiments using 8 mg/ml commercial fibrinogen and thrombin to initiate fibrinogenesis. The initial volume of the fibrin embolus was approximately 10 mm³. In the control group, which was treated with 0.25% trypsin alone, the thrombolysis rate was 0.109 mm³/min, and after 40 minutes, only 29% of the artificial fibrin embolus had been dissolved. Conversely, when microrobots were introduced alongside 0.25% trypsin, complete removal of the artificial fibrin embolus was achieved in just 17.5 minutes, with an average lysis rate of 0.57 mm³/min, reaching a peak rate of 1.147 mm³/min-nearly tenfold higher than the trypsin-only group. It was also found that without therapeutic agents, pure mechanical interaction of the microrobot causes negligible lysis. **Figure 17** depicts the results of this study. Fig. 17 shows the normalized lysis by showing the portion of undissolved fibrin embolus material as a function of the time in minutes for the set-up with the treatment with 0.25% trypsin in the presence of microrobots and a rotating magnetic field (curve 51), for the set-up with pure mechanical interaction (with microrobots and rotating magnetic field but without any thrombolytic agent; curve 53), and for the treatment of 0.25% trypsin, in the presence of the microrobots but without any rotating magnetic field (curve 52), respectively.

In an additional study, the lysis capability of the microrobots was also tested using porcine blood clots derived from blood plasma. **Figure 18** again shows the normalized lysis by showing the portion of undissolved fibrin embolus material as a function of the time in minutes, for the set-up with the treatment with 0.25% trypsin in the presence of microrobots and a rotating magnetic field (curve 55) and for the set-up with the treatment with 0.25% trypsin in the presence of microrobots but without a rotating magnetic field (curve 56). The PEGylated anisotropic microrobot with 0.25% trypsin group lysed an 8.84 mm³ blood clot in approximately 23 minutes, whereas the trypsin-only treatment, at the same concentration, dissolved only 2.4 mm³ of the clot after 30 minutes. To enable a more accurate quantification and comparison of the lytic efficacy between the microrobot group and the trypsin-only group, the normalized lysis was computed by evaluating the extent of lysis relative to baseline volume measurements post-treatment.

**Figures 19 and 20** depict the result of yet a further study by showing a microscopy image of a Y-branched system initially comprising a porcine blood clot. The Y-branched system was operatively loaded with a microrobot solution at a concentration of 0.5 mg/ml and a trypsin solution at a concentration of 0.25% (w/v). Actuation of the microrobots was achieved via application of a rotating magnetic field at a frequency of 10 Hz. Fluorescein isothiocyanate (FITC) was utilized to enhance optical contrast for visualization of the fibrin embolus within the system.

As depicted in Fig. 19, during initial actuation, individual microrobots were dispersed (shows as dots). They migrated toward the fibrin embolus under magnetic guidance. Subsequent to 20 minutes of continuous actuation, the microrobots had undergone magnetically induced dipole-dipole interactions, resulting in self-assembly into a wheel-shaped configuration, observable under microscopy as an elongated strip-like structural aggregate, well visible in Fig. 20. Concurrently, enzymatic lysis mediated by the trypsin solution achieved partial degradation of the fibrin embolus, with a reduction in cross-sectional area of approximately 50% relative to initial dimensions.

The difference between the two observed scenarios can be attributed to the passive mechanism of drug transport. Particularly within the no-reflow regions, the inefficiency of passive diffusion hampers the drug's ability to exert its lytic action effectively. Conversely, in the group utilizing microrobot's assistance, the rapid rotation of microrobots induces localized convective flow, which markedly increases drug penetration and transportation within the embolus. This enhancement increases the likelihood of drug-thrombus interactions, thereby facilitating effective thrombolysis.

To sum up, the in vitro experiments show that the synergistic action of mechanical interaction and chemical lysis yields a markedly improved thrombolysis efficiency, achieving a lysis rate 5-10 times faster than that of chemical lysis by trypsin alone. Mechanical interaction mainly consists of two parts. Firstly, the anisotropic microrobots utilize hydrodynamic coupling to transport surrounding therapeutic agents to the no-reflow region, thereby augmenting the local concentration of these agents. Secondly, they act as flow mediators by generating a convective flow field within the no-reflow region. When exposed to an out-of-plane RMF with frequency of 10 Hz, the microrobots experience a continuous magnetic torque that propels them forward. This propulsion disrupts the static flow within the no-reflow region and generate a volumetric net flow which significantly enhances the penetration and distribution of therapeutic agents into the embolus, overcoming the limitations typically imposed by passive diffusion processes.

The influence of the convective flow generated by the microrobots' rotation was experimentally confirmed using a tracer dye (Albumin-fluorescein isothiocyanate conjugate, FITC conjugate). By quantifying the fluorescence intensity of FITC within thrombi, it could be confirmed that the rotation of microrobots substantially enhances the penetration depth of rhodamine into fibrin emboli, by more than a factor 2 compared to a control group lacking the microrobot-induced convective flow (penetration depth of 2.65 mm vs. 1.23 mm). In addition, a tendency for the microrobot clusters to move towards the center of the occluded channel could be observed, resulting in an accelerated dissolution of the central region of the embolus. This is shown referring to **Figure 21****.** Based on image recognition, the periphery of the embolus 61 could be extracted, and a progressive formation of an inward-concave valley, with the depth of this depression increasing over time could be observed. In Fig. 21, the dashed arrow shows the direction of the microrobots, and the lines progressing from the top right towards the bottom left show the periphery of the embolus 61 as a function of time, see the shading scale on the right-hand side indicating the time in minutes, with the lightest shading being the initial periphery and the darkest shading the periphery after 24 minutes.

As a result of the concentration of the microrobots in the center, an area of the interface between the emboli and the therapeutic agents tendentially increases. This phenomenon not only amplifies the exposure of the embolus surface to the trypsin, thereby enhancing the lysis rate, but also mitigates potential damage to the vascular endothelium. This attenuation of damage is attributable to the aggregation behavior of the microrobots, which are drawn towards the centre of the no-reflow region. Consequently, the periphery of the embolus, particularly those segments juxtaposed to the vascular wall, undergo dissolution primarily through chemical lysis. The mechanical forces exerted by the microrobots, while enhancing lysis at the clot's center, have a comparatively diminished impact on the vascular endothelium. Thus, this synergy between chemical lysis and targeted mechanical interaction optimizes the thrombolytic process, ensuring efficacy while preserving vascular integrity.

For the treatment of other thrombotic conditions/diseases than MVO, a similar approach may be used, with the location of the injection of the microrobots and of the therapeutic agent accordingly adapted so that the transport of a therapeutic agent dispensed into the blood may reach the thrombus that obstructs a blood flow through the vessel through which the therapeutic agent is to be guided to the thrombus.

In addition or as an alternative to aiding the transport of a therapeutic agent dispensed into the blood that may reach the thrombus, by hydrodynamic coupling and/or generating a convective flow, the microrobots may also be used to directly transport a therapeutic agent. For example, a therapeutic agent may be chemically or physically bonded to a surface of the microrobots, in a manner that the bond is released when the microrobot is at the thrombus.

Also other diseases than thrombotic diseases may be treated by the microrobots according to the invention. Generally, diseases that require a targeted dispensing of a therapeutic agent to a specific location and/or a dispensing to locations with weak blood flow or flow of other body fluids, such as lymphatic fluid, may be treated, using the accordingly chosen therapeutic agent effective for treating such disease. Yet another application of the microrobots may be the targeted obliteration, by the microrobots themselves, of vessels leading to tumors or vessels to be shrunk, such as varicose veins, spider veins. In such applications, the microrobots may locomote towards the vessel to be obliterated by applying a time-dependent magnetic field, the microrobots being chosen to be of a size sufficient for them to get stuck near the target site.

## Claims

1. A microrobot, comprising:
- a plurality of magnetic particles; and
- a polymer matrix embedding the magnetic particles;
- wherein the magnetic particles and the polymer matrix are biocompatible and resorbable;
- wherein the microrobot is magnetically anisotropic; and
- wherein the microrobot has an overall anisotropic shape.

2. The microrobot according to claim 1, having a longest extension of between 3 µm and 50 µm, in particular between 5 µm and 30 µm, for example between 6 µm and 15 µm.

3. The microrobot according to claim 1 or 2, wherein the polymer matrix comprises Dextran.

4. The microrobot according to any one of the previous claims, wherein a surface of the polymer matrix is PEGylated.

5. The microrobot according to any one of the previous claims, wherein the magnetic particles are iron oxide nanoparticles having an average size of between 5 nm and 70 nm.

6. A kit of parts, comprising a plurality of microrobots according to any one of the previous claims and a thrombolytic agent to be delivered together with the microrobots and/or a catheter system for delivering the microrobots to a vessel of a human or non-human animal vessel.

7. A method of manufacturing a plurality of microrobots, the method comprising the steps of: providing a plurality of droplets of a flowable compound of magnetic particles suspended in a pre-matrix material; exposing the droplets to a magnetic field while the pre-matrix material is in a flowable state, whereby the droplets are deformed into an elongate shape with a long extension parallel to the magnetic field, because the magnetic particles re-arrange in the magnetic field; and causing the pre-matrix material to harden while the droplets are exposed to the magnetic field for the droplets to become the microrobots.

8. The method according to claim 7, comprising the further step of PEGylating the microrobots after the step of hardening.

9. The method according to claim 7 or 8, wherein the magnetic field is a rotating magnetic field with a rotation frequency of at most 20 Hz.

10. The method according to any one of claims 7-9, wherein the step of providing the droplets of the flowable compound comprises the sub-steps of providing the flowable compound and of dispersing a portion of the flowable compound in a carrier liquid that is immiscible with the flowable compound to yield an emulsion with the flowable compound as the dispersed phase and the carrier liquid as the continuous phase.

11. The method according to any one of claim 7-10, wherein the step of hardening comprises causing the pre-matrix material to harden by a polyaddition reaction.

12. A thrombolytic agent for use in a thrombolytic therapy method of a human patient or non-human animal patient, the method comprising the steps of:
- providing a plurality of microrobots, the microrobots each comprising a plurality of magnetic particles and a polymer matrix embedding the magnetic particles, wherein the magnetic particles and the polymer matrix are biocompatible and resorbable, wherein the microrobots are magnetically anisotropic, and wherein the microrobots have an overall anisotropic shape;
- administering the thrombolytic agent and the microrobots to a blood vessel in a treatment zone of the patient; and
- applying a rotating magnetic field to the treatment zone, so as to cause the microrobots to undergo a synchronous rotational movement.

13. The thrombolytic agent for use according to claim 12, wherein the step of administering comprises administering the thrombolytic agent in a flowable form directly to the blood vessel.

14. The thrombolytic agent for use according to claim 12 or 13, wherein in the step of applying the rotating magnetic field, the rotating magnetic field has a rotation frequency of between 5 Hz and 30 Hz.

15. A thrombolytic therapy method for treating a human patient or non-human animal patient in need thereof, the method comprising the steps of:
- providing a plurality of microrobots, the microrobots each comprising a plurality of magnetic particles and a polymer matrix embedding the magnetic particles, wherein the magnetic particles and the polymer matrix are biocompatible and resorbable, wherein the microrobots are magnetically anisotropic, and wherein the microrobots have an overall anisotropic shape;
- administering the thrombolytic agent and the microrobots to a blood vessel in a treatment zone of the patient; and
- applying a rotating magnetic field to the treatment zone, so as to cause the microrobots to undergo a synchronous rotational movement.
